Europäisches Patentamt

(19) European Patent Office

Office européen des brevets

(11) Publication number: **0 356 340 B1**

## EUROPEAN PATENT SPECIFICATION

(45) Date of publication of patent specification: **02.11.94** (51) Int. Cl.⁵: **A61K 9/50**, A61K 39/145

(21) Application number: **89402344.9**

(22) Date of filing: **25.08.89**

(54) **Affinity associated vaccine.**

(30) Priority: **25.08.88 US 236701**
**25.08.88 US 236702**
**23.08.89 US 397758**

(43) Date of publication of application:
**28.02.90 Bulletin 90/09**

(45) Publication of the grant of the patent:
**02.11.94 Bulletin 94/44**

(84) Designated Contracting States:
**AT BE CH DE ES FR GB GR IT LI LU NL SE**

(56) References cited:
**EP-A- 0 047 480**
**WO-A-86/05977**
**US-A- 4 148 876**
**US-A- 4 199 565**

**DATABASE/STN, vol. 105, no. 16, 1986, Columbus, OH (US); K. HASHIMOTO et al., no. 139536j&NUM;**

**DATABASE/STN, vol. 110, no. 23, 1989, Columbus, OH (US); B.J. BORMANN et al., no. 208025j&NUM;**

**VACCINE, vol. 5, June 1987; G. GREGORIADIS et al., pp. 145-150&NUM;**

(73) Proprietor: **THE LIPOSOME COMPANY, INC.**
**One Research Way**
**Princeton Forrestal Center**
**Princeton, NJ 08540 (US)**

(72) Inventor: **Popescu, Mircea C.**
**5 Parkway Avenue**
**Plainsboro, NJ 08536 (US)**
Inventor: **Recine, Marie S.**
**19 Hoffman Drive**
**Hamilton Twp., NJ 08690 (US)**
Inventor: **Alving, Carl L.**
**3 Newbold Court**
**Bethesda, MD 20817 (US)**
Inventor: **Estis, Leonard F.**
**56 Grafton Road**
**Upton, MA 01568 (US)**
Inventor: **Keyes, Lynn D.**
**56 Grafton Road**
**Upton, MA 01568 (US)**
Inventor: **Janoff, Andrew S.**
**1807 South Crescent Boulevard**
**Yardley, PA 19067 (US)**

(74) Representative: **Martin, Jean-Jacques et al**
**Cabinet REGIMBEAU**
**26, Avenue Kléber**
**F-75116 Paris (FR)**

**Description**

FIELD OF THE INVENTION

This invention concerns a vaccine against an infective agent, the vaccine comprising a liposome having an exterior and an interior and having externally disposed affinity (noncovalently) associated antigen material of at least one, preferably nonpartitioning, antigen representative of said infective agent. Also disclosed is a method of preparation and use of this vaccine.

BACKGROUND OF THE INVENTION

In the vaccine art antigens are introduced into an organism in a manner so as to stimulate an immune response in the host organism. The induction of an immune response depends on many factors among which are believed to be the chemical composition and configuration of the antigen, the potential of the immune system of the challenged organism, and the manner and period of administration of the antigen. An immune response has many aspects some of which are exhibited by the cells of the immune system, (e.g.,B-lymphocytes, T-lymphocytes, macrophages, and plasma cells). Immune system cells may participate in the immune response through interaction with antigen, interaction with other cells of the immune system, the release of cytokines and reactivity to those cytokines. Immune response is conveniently (but arbitrarily) divided into two main categories -- humoral and cell-mediated. The humoral component of the immune response includes production of immunoglobulins specific for the antigen. The cell-mediated component includes the generation of delayed-type hypersensitivity and cytotoxic effector cells against the antigen.

In some instances immune response is the result of an initial or priming dose of an antigen that is followed by one or more booster exposures to the antigen. Priming with relatively strong immunogens and liposomes is discussed in "Liposomal Enhancement of the Immunogenicity of Adenovirus Type 5 Hexon and Fiber Vaccines", Kramp, W.J. et al., Infection and Immunity, 25:771-773 (1979) and "Liposomes as Adjuvants with Immunopurified Tetanus Toxoid; the Immune Response", Davis, D. et al., Immunology Letters, 14:341-8 (1986/1987).

Ideally, an antigen will exhibit two properties, the capacity to stimulate the formation of the corresponding antibodies and the propensity to react specifically with these antibodies. Antigens bear one or more epitopes which are the smallest part of an antigen recognizable by the combining site of an antibody.

In particular instances antigens or fractions of antigens or with particular presenting conditions the immune response precipitated by the desired antigen is inadequate or nonexistent and insufficient immunity is produced. This is particularly the case with peptide or other small molecules used as antigens.

In such cases the vaccine art recognizes the use of substances called adjuvants to potentiate an immune response when used in conjunction with an antigen or immunogen. Adjuvants are further used to elicit immune response sooner, or a greater response, or with less antigen or immunogen or to increase production of certain antibody subclasses that afford immunological protection, or to enhance components of the immune response (e.g., humoral, cellular). Liposomal vaccines and adjuvancy are further discussed in European Patent Application Ser. No. 89402343.1 to Popescu et al., filed on date even herewith the teachings of which are incorporated herein by reference.

Known adjuvants are Freund's Adjuvants (and other oil emulsions), Bortedella Pertussis, Lipid A (the glycophospholipid moiety of lipopolysaccharide found in Gram-negative bacteria), aluminium salts (and other metal salts), Mycobacterial products (including muramyl dipeptides), and liposomes. As used herein the term "adjuvant" will be understood to mean a substance or material administered together or in conjunction with an antigen which increases the immune response to that antigen. Adjuvants may be in a number of forms including emulsion (e.g., Freund's adjuvant) gels (aluminium hydroxide gel) and particles (liposomes) or as a solid material.

It is believed that adjuvant activity can be affected by a number of factors. Among such factors are (a) carrier effect, (b) depot formation, (c) altered lymphocyte recirculation, (d) stimulation of T-lymphocytes, (e) direct stimulation of B-lymphocytes and (f) stimulation of macrophages.

With many adjuvants adverse reactions are seen. In some instances adverse reactions include granuloma formation at the site of injection, severe inflammation at the site of injection, pyrogenicity, adjuvant induced arthritis or other autoimmune response, or oncogenic response. Such reactions have hampered the use of adjuvants such as Freund's adjuvant.

In particular embodiments liposome adjuvants are utilized. U.S. Patent No. 4,053,585 issued October 17, 1977 to Allison et al. states that liposomes of a particular charge are adjuvants.

Other substances such as immunomodulators (e.g., cytokines such as the interleukins) may be combined in adjuvants as well.

Humoral immune response may be measured by many well known methods. Single Radial Immunodifussion Assay (SRID), Enzyme Immunoassay (EIA) and Hemagglutination Inhibition Assay (HAI) are but a few of the commonly used assays.

EIA, also known as ELISA (Enzyme Linked Immunoassay), is used to determine total antibodies in a sample. The antigen is adsorbed to the surface of a microtiter plate. The test serum is exposed to the plate followed by an enzyme linked immunogloublin, such as IgG. The enzyme activity adherent to the plate is quantified by any convenient means such as spectrophotometry and is proportional to the concentration of antibody directed against the antigen present in the test sample.

Tests to measure cellular immune response include determination of delayed-type hypersensitivity or measuring the proliferative response of lymphoctyes to target antigen.

Liposomes are completely closed lipid bilayer membranes containing an entrapped aqueous volume. Liposomes may be unilamellar vesicles (possessing a single bilayer membrane ) or multilameller vesicles (onion-like structures characterized by multiple membrane bilayers, each separated from the next by an aqueous layer). The bilayer is composed of two lipid monolayers having a hydrophobic "tail" region and a hydrophilic "head" region. The structure of the membrane bilayer is such that the hydrophobic (nonpolar) "tails" of the lipid monolayers orient toward the center of the bilayer while the hydrophilic "head" orient towards the aqueous phase.

The original liposome preparation of Bangham, et al. (J. Mol. Biol., 1965, 13:238-252) involves suspending phospholipids in an organic solvent which is then evaporated to dryness leaving a phospholipid film on the reaction vessel. Next, an appropriate amount of aqueous phase is added, the mixture is allowed to "swell," and the resulting liposomes which consist of multilamellar vesicles (MLVs) are dispersed by mechanical means. This technique provides the basis for the development of the small sonicated unilamellar vesicles described by Papahadjopoulos et al. (Biochem. Biophys. Acta., 1968, 135:624-638), and large unilamellar vesicles.

Unilamellar vesicles may be produced using an extrusion apparatus by a method described in Cullis et al., PCT Application No. WO 86/00238, published January 16, 1986, entitled "Extrusion Technique for Producing Unilamellar Vesicles" incorporated herein by reference. Vesicles made by this technique, called LUVETS, are extruded under pressure once or a number of times through a membrane filter. LUVETs will be understood to be included in the term "unilamellar vesicle".

Another class of liposomes are those characterized as having substantially equal lamellar solute distribution. This class of liposomes is denominated as stable plurilamellar vesicles (SPLV) as defined in U.S. Patent No. 4,522,803 to Lenk, et al., monophasic vesicles (MPVs) as described In U.S. Patent no. 4,588,578 to Fountain, et al. and frozen and thawed multilamellar vesicles (FATMLV) wherein the vesicles are exposed to at least one freeze and thaw cycle; this procedure as described in Bally et al., PCT Publication No. 87/00043, January 15, 1987, entitled "Multilamellar Liposomes Having Improved Trapping Efficiencies". U.S. Patent No. 4,721,612 to Janoff et al. describes steroidal liposomes for a variety of uses. The teachings of these references as to preparation and use of liposomes are incorporated herein by reference.

Lipids of net negative charge are well known in the art and include for example, phosphatidyserine, phosphatidic acid, and phosphatidylglycerol. Lipids of net positive charge are well known in the art and include for example, aminodiglycerides, glyceridecholine, sterylamine, trimetylsterylamine, and dioctadecyl trimethyl ammonio propane. In general any bilayer forming amphiphils which has a charged hydrophilic moiety may be used.

In addition, lipid charge may be manipulated by a number of methods well known in the art, such as by linking the lipid to a moiety of appropriate net charge. For example, the neutral lipid cholesterol may be linked to succinic acid (negative charge) to yield cholesterol hemisuccinate (CHS) of negative charge. The tris(hydroxymethyl)aminomethane form of CHS is designated $CHS_{tris}$ and its application to liposomes is more fully discussed in U.S. Patent No. 4,721,612 the teachings of which are incorporated herein by reference.

Gregoriadis et al, vaccin vol.5 June 1987, 145-150 discusses the use of PC/cholesterol MLV liposomes in which the antigen is chemically surface linked to the liposomes by diazotization covalent coupling.

## Summary of the Invention

This invention includes a composition comprising a liposome in noncovalent association with an externally disposed antigen and in one embodiment further comprising adjuvant or further comprising aluminum hydroxide or Lipid A. Preferred antigens are nonpartitioning. In some embodiments the antigen is hydrophilic or lipophilic. Variously the affinity association is noncovalent association and the like such as electrostatic, hydrophilic, hydrogen bonding, or other bonding related to van der Waals forces such as configurational stickiness. Liposomes of this invention may be unilamellar or multilamellar.

In particular applications liposomes may comprise cholesterol hemisuccinate, phosphatidylserine, phosphatidic acid, or phosphatidylglycerol as well as aminodiglyceride, glyceridecholine, stearylamine, trimethylstearylamine, dioctadecyl trimethylammonio derivatives (e.g., 1,2 bis(oleoyloxy)-3-dioctadecyl trimethylammonio propane -- "DOTAP") or any bilayer forming amphiphile having a charged hydrophilic moiety.

Useful antigens include HIV or portion thereof with particular reference to PB1. Antigen includes peptide, glycopeptide or glycoprotein. In particular applications the antigen is influenza or fragments thereof, herpes or fragments thereof, haemophilus B or fragments thereof or malaria or fragments thereof. Antigens also include isolated or bioengineered fragments of viruses, bacteria, cancer cells, humoral cells and body fluid components.

In another embodiment the invention includes a method of producing a vaccine composition comprising a liposome in affinity (noncovalent) association with an externally disposed and preferably nonpartitioning antigen comprising contacting, in an aqueous solution, an antigen and a liposome. This method compris said bilayer forming material of reciprocal affinity to said antigen, such that the antigen and the liposome form an affinity association; optionally the process may further include removing non-affinity associated antigen.

In the practice of this method the affinity between antigen and liposome is electrostatic or hydrogen bonding or is configurational stickiness. Additionally by this method the liposome is of net negative charge and the antigen of net positive charge or the liposome is of net positive charge and the antigen of net negative charge. In the practice of this method in one embodiment the liposome comprises CHS. In another embodiment the antigen comprises PB1. In particular applications the liposomes are subjected to shearing force.

This invention yet further includes a method of inducing an immune response in an animal, including a human, comprising administering to said animal a therapeutically effective amount of a composition comprising a liposome in noncovalent affinity association with an externally disposed preferably nonpartitioning antigen. The method can further utilize adjuvant such as aluminium hydroxide or Lipid A. Variously by this method antigen is hydrophilic or lipophilic. Further by this method the affinity is electrostatic, hydrogen bonding or configurational stickiness.

In the practice of this method of treatment in various embodiments the liposome comprises cholesterol hemisuccinate, phosphatidylserine, phosphatidic acid or phosphatidylglycerol as well as aminodiglyceride, glyceridecholine, stearylamine, trimethylstearylamine, dioctadecyl trimethylammonio derivatives or any bilayer forming amphiphile having a charged hydrophilic moiety. Antigens can comprise HIV or portions thereof particularly or PB1. Variously antigens are noted to be protein, peptide, glycopeptide or glycoprotein, polypeptide, or poly(amino acid) and will be termed, collectively, "peptide". Particularly noted as antigens are influenza or fragments thereof, herpes or fragments thereof, haemophilus B or fragments thereof or malaria or fragments thereof as well as isolated or bioengineered fragments of viruses, bacteria, cancer cells, humoral cells and body fluid components.

Detailed Description of the Invention

For clarity, in the discussion of this invention the following definitions will be used:

"Adjuvant" shall mean a substance or material to potentiate an immune response when used in conjunction with an antigen or immunogen. Adjuvants are further used to elicit immune response sooner, or a greater response, or with less antigen.

"Antigen" shall mean a substance or material that is recognized specifically by an antibody and/or combines with an antibody. Particular note is made of both natural and bioengineered antigens such as peptides, glycopeptides and glycoproteins. Specific antigens include antivirals such as herpes, hepatitis, rabies, parainfluenza, measles, mumps, respiratory syncytial virus; antibacterials such as pneumonia, haemophilis B, staphylococcus, meningococcus, Neisseria gonorrhea; and protozoa such as malaria or fragments thereof.

"Epitope" shall mean the smallest part of an antigen recognizable by the combining site of an immunoglobulin.

"Externally disposed" shall, in referring to antigen or immunogen, mean, positioned by an "affinity association" so as to bear an epitope external to the outermost lamella of an associated liposome. Included are epitopes ionically associated with the liposome, nonpartitioned into the outermost lamellae, and with epitope exposed.

"Affinity association" shall mean noncovalent intermolecular associations such as electrostatic association, hydrogen bonding, and configurational stickiness.

"Configurational stickiness" shall be understood to mean physical parameters that facilitate immobilization of antigen or immunogen externally on a liposome such as van der Waals forces.

"Nonpartitioning" refering to antigen or immunogen refers to hydrophilic immunogens and those lipophilic immunogens the epitopes of which are not substantially incorporated into the outermost lamella of a liposome. Those lipophilic immunogens that can be separated from liposomes by physical manipulation such as by dialysis, charge manipulation or other equilibrium based separations are deemed to be not substantially incorporated into the lamellae and nonpartitioning.

"Immune response" shall mean a specific response of the immune system of an animal to antigen or immunogen. Immune response may include the production of antibodies.

"Immunity" shall mean a state of resistance of a subject animal to an infecting organism or substance. It will be understood that infecting organism or substance is defined broadly and includes parasites, toxic substances, cancers and cells as well as bacteria and viruses. A Therapeutically Effective Immunization Course will produce immune response to protect the organism against challenging antigen.

"Immunization conditions" shall mean factors which affect an immune response including amount and kind of antigen or adjuvant delivered to a subject animal, method of delivery, number of inoculations, interval of inoculation, the type of subject animal and its presenting condition.

"Immunization dose" shall mean the amount of antigen or immunogen needed to precipitate an immune response. This amount will vary with the presence and effectiveness of various adjuvants. This amount will vary with the animal and immunogen or antigen or adjuvant but will generally be between about 0.1ug/ml or less to about 500ug per inoculation. The immunization dose is easily determined by methods well known to those skilled in the art, such as by conducting statistically valid host animal immunization and challenge studies. See, for example, Manual of Clinical Immunology, H.R. Rose and H. Friedman, American Society for Microbiology, Washington, D.C. (1980). In some instances several immunization doses including booster doses will be administered to provide immunity.

"Immunogen" shall mean a substance or material (including antigens) that is able to induce an immune response alone or in conjunction with an adjuvant. This will generally be a protein, peptide, polysaccharide, nucleoprotein, lipoprotein, synthetic polypeptide, or hapten liked to a protein, peptide, polysaccharide, nucleoprotein, lipoprotein or synthetic polypeptide or other bacterial, viral or protozoal fractions. It will be understood that "immunogen" includes substances which do not generate an immune response (or generate only a therapeutically ineffective immune response) unless associated with an adjuvant (e.g., small peptides) which will be referred to on "adjuvant-obligatory" immunogens.

"Infective agent" shall mean an disease causing agent including fungi, bacteria, viruses and parasites.

"Mimetic", in relation to antigens, immunogens and epitopes, shall refer to a moiety (either natural or synthetic) that duplicates by structure, accessibility or reactivity the response of B-cell immuno-receptors to the subject antigen in native state configuration or epitope in native state configuration.

"Native state configuration" shall mean that organization of a moiety as it is when present in situ in its usual condition, to be distinguished from non-native state configuration (denatured) wherein the moiety is altered as to immuno-reactivity from that of the in situ organization.

"Vaccine" shall mean a pharmaceutical formulation which induces immune response or immunity in a subject animal.

Antigens or immunogens may have a net positive or negative charge, or be neutral. The net charges are easily determined by a number of techniques well known in the art. For peptides the net charge may be assessed through determination of the isoelectric point. Peptides of isoelectric points of about 5 or less have net negative charge and the peptides are soluble in basic solution. Isoelectric points of about 8 or more indicate a net positive charge and the peptides are soluble in acid solution. Isoelectric points from 5 to 8 indicate generally neutral peptides (from slightly acidic to slightly basic). The determination of isoelectric points is well known in the art and is discussed in "Relationship Between in vivo Degradative Rates and Isoelectric Points of Proteins", Dice, et al., Proc. Natl. Acid. Sci. USA, 72:3895-97 (1975) the teachings of which are incorporated herein by reference.

Net charges on peptides can be manipulated by a number of methods well known in the art including adding charge bearing amino acids or amino acid segments. By way of example lysine and arginine add positive charge while aspartic and glutamic acid add negative charge. It is important to note in any manipulation of antigens or immunogens including addition of amino acids or exposure to acidic or basic solutions or temperature variation or light that conditions must not be such as to compromise immunogenicity or native state configuration.

A particularly useful immunogen is the PB1 fraction of the HIV 1 virus (Repligen Corporation, Cambridge, MA). This fraction is characterized in "HTLV-III/LAV-Neutralizing Antibodies of an E. coli-Produced Fragment of the Virus Envelope", Putney, et al., Science, 234:1392-5 (1986) the teachings of which are incorporated herein by reference. PB1 peptide is soluble in aqueous solution containing solubilizing substances such as urea and detergents and was supplied in such solution. Such solubilization substances were incompatible with pharmaceutical preparations and a dialysis process was utilized to remove them.

Antigene or immunogens which partition into the liposome lamellae such as melanoma antigen in CHS liposomes may not yield a sufficient immunogenic response without repeated inoculation and additional adjuvant. Without being bound by any particular theory it is believed that this partitioning results in the limitation of exposure of epitopes externally to the adjuvant liposomes. Modification (e.g., conjugation with a nonpartitioning moiety) or such an antigen or immunogen while preserving native state configuration acts so as to prevent partitioning. In addition it can be advantageous to utilize both affinity associated antigen and entrapped antigen in a particular composition, vaccine or dosage form.

A preferred vaccine of this invention is a liposome organized so as to have in immunogen in electrostatic association with the exterior of said liposome. The preferred liposome may be multilamellar (e.g., multilamellar vesicles or stable plurilamellar vesicles). In a particular embodiment the vaccine contains externally disposed antigen or immunogen in such electrostatic association.

In some embodiments the most successful immunogens will be those purified from (or duplicative or mimetic of) the infective agent while maintaining immunogen native state configuration. It is understood that some vaccines are prepared without resort to living infective organisms due to the potential for contamination of the final vaccine. It is thus useful to produce immunogens synthetically (including bioengineering) such as through techniques of recombinant DNA technology, recombinant RNA technology or other synthesis in host cells, but it is preferable that even synthetically produced immunogens be of native state configuration.

Selection of lipid and antigen of reciprocal affinity is simply accomplished by those skilled in the art. Depending upon the type of noncovalent affinity association applicable various association techniques are used. For hydrogen bonding the elevation of temperature of the aqueous solution suspending antigen and liposomes above the transition temperature of the affinity bonded entities is effective. For van der Waals, or electrostatic or configurational stickiness admixing of the antigen and liposomes in aqueous solution is sufficient, but is to electrostatic affinity the liposome and antigen are of opposite charge.

Degree of affinity association of nonpartitioning antigen or immunogen is simply determined by methods well known in the art. By one method the relative attachment and nonattachment is determined by centrifuging the liposomes and antigen or immunogen admixture and then determining the amount of antigen or immunogen in the supernatant. The amount not in the supernatant being a measure of the amount affinity attached.

Also useful is priming in generation of the immune response. A vaccine may generate a weak immune response that is not greatly potentiated on secondary challenge. However, after a first administration of the adjuvant-associated immunogen, a substantial immune response is generated upon application of a second inoculation of the adjuvant free immunogen in solution.

The efficacy and utility of this invention is disclosed in Tables 1 and 2.

Table 1 presents the data of an experiment in which $CHS_{tris}$ formulation was compared with other liposome formulations.

Liposomes made of $CHS_{tris}$ (Table 1, group F) were prepared by multilamellar vesicles (MLV) procedure. This formulation involves the coating of the surface of negatively charged liposomes with the positively charged antigen.

Liposomes made of dimyristoylphosphatidylcholine/cholesterol (DMPC/CHOL) were prepared using either stable plurilamellar vesicle (SPLV) procedure (D-W, D) or monophasic vesicle (MPV) procedure (D-E, D-T). The following procedures A-E are for illustrative purposes only and are not within the scope of the invention as claimed.

A. SPLV Liposome D-W

DMPC (80 mg) and cholesterol (20 mg) in 1 ml chloroform were rotoevaporated under vacuum to a dry film and further solubilized in 5 ml ether + 0.5 ml ethanol at 40°C for a few seconds until the solution was clear. The PB1 antigen (100 ug) in 0.5 ml 1% acetic acid was added to this solution and the resulting mixture was sonicated under a stream of $N_2$ at 40°C for about 1 minute until dryness under $N_2$ stream (2-3 minutes). The resulting dry material was resuspended in 10 ml phosphate buffered saline minus $Ca^{++}$ and $Mg^{++}$ ($KCl$ 2g/L, $KH_2PO_4$ 2 g/L, NaCL 80 g/L, $NaPO_4$ $7H_2O$ 21.6 g/L) ("PBS minus") to a milky suspension which was then centrifuged at 10,000 rpm for 10 minutes at 4°C. The pellet of liposomes was resuspended in 2 ml PBS minus to a final concentration of 50 mg lipid/ml.

B. SPLV. Liposome D

This liposome was prepared as described above for the D-W liposome except that the centrifugation step was omitted. Subsequently the material dried by $N_2$ stream was resuspended in 2 ml PBS minus to a final concentration of 50 mg lipid and 50 ug antigen/ml.

C. MPV. Liposome D-E

Cholesterol (20 mg) was dissolved in 1 ml ethanol containing 80 mg solubilized DMPC by brief heating to 40°C (10-20 sec.) and stirring. The solution of antigen (1 ml) was prepared separately by mixing 100 ug PB1 in 0.13 ml 1% acetic acid and 0.87 ml ethanol and immediately mixed with the lipid solution. The mixture was rotoevaporated under vacuum at 40°C and the resulting material was resuspended in 2 ml PBS minus by sonication for 10 seconds at 40°C to a final suspension of 50 mg lipid and 50 ug antigen/ml.

D. MPV. Liposome D-T

This liposome was prepared as described above for the D-E liposome except that the ethanol was replaced by Tert-butyl-alcohol (TBA).

E. Liposome, H-D mixture

This preparation was done by mixing 0.5 ml of SPLV, liposome D made of DMPC/CHOL and 0.5 ml of empty MLV liposome H made of $CHS_{tris}$ as described below. The mixture contained 75 mg lipids (50 mg $CHS_{tris}$, 25 mg DMPC/CHOL) and 25 ug PB1 antigen/ml.

F. MLV, Liposome H

Empty $CHS_{tris}$ liposomes were prepared by hydration of 100 mg $CHS_{tris}$ with 1 ml of PBS minus for 2 hr. at room temperature and intermittent vigorous vortex-mixing. Separately, a fresh dialysate of PB1 antigen in 1% acetic acid was diluted in PBS minus at 100 ug antigen/ml and 0.5 ml of this solution was immediately mixed with 0.5 ml of empty $CHS_{tris}$ liposomes to allow affinity association between vesicles and antigen. The mixture contained 50 mg lipid and 50 ug antigen/ml.

G. Control of Complete Freund Adjuvant (CFA) and dialyzed (d) antigen

Equal 1 ml volumes of complete Freund's adjuvant (CFA) oil and PB1 dialyzed against 1% acetic acid and further diluted in 1% acetic acid to 100 ug antigen/ml, were mixed thoroughly and emulsified by repeated passage through a 16G needle connecting two 5 ml syringes. This emulsion contained 50 ug antigen/ml.

H. Control of CFA and non-dialyzed antigen

This emulsion was prepared as described above except that PB1 antigen was not dialyzed.

I. Control of non-dialyzed antigen in solution

PB1 at a concentration of 0.77 mg/ml in a 50 mM phosphate buffer, pH 6.8, containing 2 mM EDTA, 10 mM DTT and 8 M urea was diluted in PBS minus to 50 ug/ml.

J. Control of dialyzed antigen in solution

Stock PB1 solution (0.77mg/ml in a 50 mM phosphate buffer, pH 6.8, containing 2 mM EDTA, 10 mM dithiothneitrol and 8 m urea) was dialyzed against 1% acetic acid (pH 2.5) and the dialyzed antigen diluted before use in PBS minus at 50 ug/ml.

Immunization: Balb/C (Jakcson), 6-8 weeks old female mice were inoculated intramuscularly ("IM") at day 0, 15, 35 and blood was taken at day 0, 14, 28, 49, 63 and 79. The dose of antigen was 5 ug/0.1 ml/inoculum except group E which received 2.5 ug.

Determinition of humoral immune response: PB1 specific antibody (IgG and IgM) was determined by a standard ELISA procedure.

Data presented in Table 1 indicated that the adjuvant effect provided by CHS liposomes with affinity associated immunogen (group F) was similar with that provided by complete Freund's adjuvant "CFA" (groups G and H), a potent but highly toxic adjuvant.

Out of six liposomal formulations, five induced a positive response in all mice tested 49 days or more after primary inoculation, indicating an enhancement by liposomes of the secondary IgG memory response and involvement of helper T-cells. The magnitude of the response, however, was highly dependent on the nature of liposome formulation and varied from low (groups A, B, C), medium (group E) to high (group F).

Since the dose of antigen in group E was 2.5 ug/0.1 ml/inoculum (rather than 5 ug) the adjuvant effect provided by this formulation was probably underestimated. Both the original and acid dialyzed PB1 in PBS minus were weak immunogens (group I and J). In contrast, the CHS liposome affinity associated preparation (group F) induced a high antibody response and in addition no or minimal reaction was observed at the site of inoculation.

TABLE 2

In order to compare the effect of CHS affinity associated liposomes with that of Alum, a standard adjuvant used in vaccines for humans, groups of five mice were immunized with 20, 5, 1.25 and 0.31 ug PB1 in either Alum or CHS formulation.

The amount of Alum was kept constant at 0.4 mg/ml of physiological saline (USP). The CHS liposome-PB1 formulations were prepared as described previously (group F, table 3). A formulation containing 50 ug antigen/ml was further diluted in a suspension of empty CHS liposomes in PBS minus such that the amount of lipids was kept constant at 50 mg/ml and the antigen decreased to 2.5, 1.25 and 0.31 ug/ml. Another CHS formulation at 50 mg lipid and 20 ug antigen was subjected to shearing force via vortexing and sonicating at low energy intermittently for 2 hours at room temperature in order to achieve a suitable suspension.

The results (Table 2) indicated that the CHS liposome with affinity associated immunogen was a much stronger adjuvant than Alum. The difference in immune titers were in general higher than one order of magnitude with the exception of lowest dose (0.31 ug PB1) in which the response remained low.

In addition, the comparison between CHS formulation and CFA formulation both at 1.25 ug antigen reinforced the conclusion of the previous experiment (Table 1) indicating a higher antibody response by CHS affinity associated liposomes than by CFA.

Vaccines are conveniently administered in a dosage form. A "dosage form" will be understood to mean any pharmaceutically form of administering a vaccine including subcutaneous, oral, intramuscular, and ocular administration and utilizing vaccines in live, attenuated or synthetic or bioengineered or partial forms along with adjuvants and optionally immunomodulators such is cytokines. The combinations of the foregoing elements are prepared so that the dosage form is adapted to produce a therapeutically effective immune response in the subject animal including a human as easily and effectively as possible.

The dosage forms including liposomal dosage forms resulting from the method of the present invention can be used therapeutically in mammals, including a human, in the treatment of infections or conditions which require the delivery of immunogen in its bioactive form. Such conditions include but are not limited to disease states such as those that can be treated or prevented with vaccines.

Dosage forms also include use of adjuvant as well as vaccine incorporated into gel such as aluminum gels, lipids such as Lipid A, liquid crystals, powders, precipitates and solutions. In particular embodiments the dosage form can be a unit dosage form configured and adapted to a single administration.

The mode of administration of the dosage form may determine the sites and cells in the organism to which the dosage form will be delivered. The dosage forms including liposomal dosage forms of the present invention can be administered alone but will generally be administered in admixture with a pharmaceutical carrier selected with regard to the intended route of administration and standard pharmaceutical practice. The dosage forms may be injected intramuscularly, subcutaneously or intradermally. The dosage forms may also be administered via oral routes. For parenteral administration, they can be used, for example, in the form of a sterile aqueous solution which may contain other solutes, for example, enough salts or glucose to make the solution isotonic. Other uses, depending upon the particular properties of the preparation, may be envisioned by those skilled in the art.

For administration to humans in the preventive or curative treatment of disease states responding to vaccine based therapy, the prescribing physician will ultimately determine the appropriate therapeutically effective dosage for a given human subject, and this can be expected to vary according to the age, weight, and response of the individual is well as the nature and severity of the patient's disease.

EXAMPLE 1

Affinity Attachment of Antigen to Liposome

PB1 is a positively charged peptide and thus dialysis of PB1 from stock solution was performed against a 1% acetic acid at a pH of 2.5. Upon dialysis the PB1 remained soluble for several hours in 1% acetic acid. It was noted that, were the pH to approach neutral pH, the PB1 antigen would rapidly precipitate. Affinity attachment of the PB1 to liposomes was accomplished by utilizing a liposome of reciprocal (here negative) charge. $CHS_{tris}$ liposomes having a negative charge were used, suspended in neutral (pH 7.2) buffer and then mixed with the PB1 in 1% acetic acid solution. Care was taken to avoid $CHS_{tris}$ precipitation as $CHS_{tris}$ liposome suspension is rapidly precipitated by solutions below about pH 5. Therefore in order to maintain the integrity of the liposome suspension, upon addition of the PB1 in acetic acid solution the pH of the mixture was maintained above 5. Affinity association occured upon mixing of liposomes and antigen.

TABLE 1

| SERUM ANTIBODY TITERS AFTER IMMUNIZATION OF BALB/C MICE WITH LIPOSOMAL PB1 FORMULATIONS | | | | | |
|---|---|---|---|---|---|
| Group and Formulation | Geometric Main Titer (EIA Units/ml) | | | | |
| | Day 14 | Day 28 | Day 49 | Day 63 | Day 79 |
| A. Liposome D-W | - a/ (0/5) | - (0/5) | 66.08 (5/5) | 140.85 (5/5) | 74.00 (4/5) |
| B. Liposome D | - (0/5) | - (0/5) | 144.47 (5/5) | 95.68 (5/5) | 70.77 (4/5) |
| C. Liposome D-E | - (0/5) | 64.62 (4/5) | 113.00 (5/5) | 154.21 (4/4) | 180.90 (4/4) |
| D. Liposome D-T | - (0/5) | - (0/5) | - (0/5) | 35.00 (1/5) | - (0/5) |
| E. Liposome H-D | - (0/5) | 20.49 (4/5) | 663.15 (5/5) | 640.55 (4/4) | 618.41 (4/4) |
| F. Liposome H | - (0/5) | 1,135.78 (5/5) | 6,593.49 (4/4) | >11,465.31 (4/4) | 6,104.65 (3/3) |
| G. CFA-PB1 | - (0/5) | 1,138.58 (5/5) | >11,329.05 (5/5) | >11,465.31 (4/4) | 14,610.05 (4/4) |
| H. CFA-PB1 | - (0/5) | 321.03 (5/5) | 2,253.23 (5/5) | 2,875.41 (5/5) | 3,134.13 (5/5) |
| I. PB1 | - (0/5) | - (0/5) | 300.00 (1/5) | 43.47 (2/5) | - (0/4) |
| J. PB1 | - (0/5) | - (0/5) | 54.77 (2/5) | 94.87 (2/5) | 60 (1/4) |

a/ Number of responding mice per group. Mean titer was calculated from the individual titers of the responding mice only.

TABLE 2

| SERUM ANTIBODY TITERS AFTER IMMUNIZATION OF BALB/C MICE WITH VARIOUS DOSES OF PB1 IN LIPOSOMAL AND ALUM FORMULATIONS | | | | |
|---|---|---|---|---|
| Formulation | Dose (ug) | Geometric Mean Titer (EIA Units/ml) | | |
| | | Day 14 | Day 28 | Day 49 |
| Alum | 20.0 | - a/ (0/4) | 161.34 (4/4) | 1,212.31 (4/4) |
| Liposome | 20.0 | 21.79 (2/4) | 2,411.18 (4/4) | 4,355.87 (4/4) |
| Alum | 5.0 | 0 (0/4) | 77.31 (5/5) | 349.27 (5/5) |
| Liposome | 5.0 | 38.73 (2/5) | 931.97 (5/5) | 7,949.76 (5/5) |
| Alum | 1.25 | - (0/5) | - (0/5) | 79.06 (2/5) |
| Liposome | 1.25 | - (0/5) | 152.02 (4/5) | 917.78 (4/5) |
| Control (PB1 | 1.25 | - (0/5) | - (0/5) | - (0/5) |
| Control (CFA) | 1.25 | - (0/5) | 38.91 (4/5) | 365.73 (5/5) |
| Alum | 0.31 | - (0/5) | 27.00 (1/5) | 30.00 (1/5) |
| Liposome | 0.31 | - (0/5) | - (0/5) | 42.17 (3/5) |

a/ Number of responding mice per group. Mean titer was calculated from the individual titers of the responding mice only.

**Claims**

**Claims for the following Contracting States : AT, BE, CH, DE, FR, GB, IT, LI, LU, NL, SE**

1. A vaccine composition comprising an adjuvant liposome in electrostatic affinity association with an externally disposed antigen, said liposome comprising a bilayer-forming amphiphile having a net charged hydrophilic moiety.

2. The composition of claim 1 further comprising an additional adjuvant.

3. The composition of claim 2 wherein said adjuvant comprises aluminum hydroxide or Lipid A.

4. The composition of claim 1, 2 or 3 wherein the antigen is hydrophilic.

5. The composition of any one of claims 1 to 4 wherein the bilayer-forming amphiphile is of net negative charge and is selected from the group consisting of negatively charged forms of cholesterol hemisuccinate, phosphatidylserine, phosphitidic acid, and phosphatidylglycerol.

6. The composition of any one of claims 1 to 4 wherein the bilayer-forming amphiphile is of net positive charge and is selected from the group consisting of positively charged forms of aminodiglyceride, glyceridecholine, stearylamine, trimethylstearylamine and dioctadecyl trimethylammonio derivatives.

10

EP 0 356 340 B1

7. The composition of any one of claims 1 to 6 wherein the antigen is nonpartitioning.

8. The composition of any one of claims 1 to 5 wherein the antigen comprises an HIV virus or a fraction or fragment thereof.

9. The composition of claim any one of claims 1 to 5 wherein the antigen comprises the PB1 fraction of HIV 1 virus.

10. The composition of any one of claims 1 to 7 wherein the antigen comprises a peptide.

11. The composition of any one of claims 1 to 7 wherein the antigen comprises an influenza virus fraction or a fragment thereof.

12. The composition of any one of claims 1 to 7 wherein the antigen comprises a herpes virus fraction, a haemophilus B bacteria fraction or a malaria sporozoite fraction, or a fragment of any of these.

13. A method of producing a vaccine composition comprising an adjuvant liposome in electrostatic affinity association with an externally disposed antigen, said method comprising the step of contacting in an aqueous solution a) a liposome comprising a bilayer-forming amphiphile having a net charged hydrophilic moiety, and b) an antigen, such that the antigen and the liposome form an electrostatic affinity association.

14. The method of claim 13 further comprising removing non-affinity associated antigen.

15. The method of claim 13 or 14 wherein the antigen is nonpartitioning.

16. The method of any one of claims 13 to 15 wherein the bilayer-forming amphiphile is of net positive charge and the antigen has a hydrophilic moiety.

17. The method of any one of claims 13 to 15 wherein the bilayer-forming amphiphile is of net negative charge and the antigen has a hydrophilic moiety.

18. The method of Claim 17 wherein the bilayer-forming amphiphile is cholesterol hemisuccinate.

19. The method of Claim 17 or 18 wherein the antigen comprises the PB1 fraction of HIV 1 virus.

**Claims for the following Contracting States : ES, GR**

1. A method of producing a vaccine composition comprising an adjuvant liposome in electrostatic affinity association with an externally disposed antigen, said method comprising the step of contacting in an aqueous solution a) a liposome comprising a bilayer-forming amphiphile having a net charged hydrophilic moiety, and b) an antigen such that the antigen and the liposome form an electrostatic affinity association.

2. The method of claim 1 further comprising removing non-affinity associated antigen.

3. The method of claim 1 or 2 wherein the antigen is nonpartitioning.

4. The method of any one of claims 1 to 3 wherein the bilayer-forming amphiphile is of net positive charge and the antigen has a hydrophilic moiety.

5. The method of any one of claims 1 to 3 wherein the bilayer-forming amphiphile is of negative charge and the antigen was a hydrophilic moiety.

6. The method for preparing a composition of claims 1 to 5 further adding an additional adjuvant.

7. The method of claim 6 wherein said adjuvant comprises aluminium hydroxide or lipid A.

8. The method of any one of claims 1 to 7 wherein the bilayer-forming amphiphile is of net negative charge and is selected from the group consisting of negatively charged forms of cholesterol hemisuccinate, phosphatidylserine, phosphatidic acid, and phosphatidylglycerol.

9. The method of any one of claims 1 to 7 wherein the bilayer-forming amphiphile if of net positive charge and is selected from the group consisting of positively charged forms of aminodiglyceride, glyceridecholine, stearylamine, trimethylstearylamine and dioctadecyl trimethylammonio derivatives.

10. The method of claim 8 wherein the bilayer-forming amphiphile is cholesterol hemisuccinate.

11. The method of any one of claims 1 to 9 wherein the antigen comprises an HIV virus or a fraction or fragment thereof.

12. The method of claim 11 wherein the antigen comprises the PB1 fraction of HIV 1 virus.

13. The method of any one of claims 1 to 10 wherein the antigen comprises a peptide.

14. The method of any one of claims 1 to 10 wherein the antigen comprises an influenza virus fraction or a fragment thereof.

15. The method of any one of claims 1 to 10 wherein the antigen comprises a herpes virus fraction, a haemophilus B bacteria fraction or a malaria sporozoite fraction, or a fragment of any of these.

**Patentansprüche**

**Patentansprüche für folgende Vertragsstaaten : AT, BE, CH, DE, FR, GB, IT, LI, LU, NL, SE**

1. Impfstoffzusammensetzung, die ein Adjuvans-Liposom in elektrostatischer Affinitätsassoziierung mit einem außen angeordneten Antigen umfaßt, wobei das Liposom ein Bilayer-bildendes Amphiphiles umfaßt, des einen hydrophilen Teil mit Netto-Ladung hat.

2. Zusammensetzung nach Anspruch 1, die weiterhin ein zusätzliches Adjuvans umfaßt.

3. Zusammensetzung nach Anspruch 2, worin das Adjuvans Aluminiumhydroxid oder Lipid A umfaßt.

4. Zusammensetzung nach Anspruch 1, 2 oder 3, worin das Antigen hydrophil ist.

5. Zusammensetzung nach einem der Ansprüche 1 bis 4, worin das Bilayer-bildende Amphiphile eine negative Netto-Ladung hat und aus der Gruppe ausgewählt ist, die aus negativ geladenen Formen von Cholesterin-hemisuccinat, Phosphatidylserin, Phosphatidsäure and Phosphatidylglycerin besteht.

6. Zusammensetzung nach einem der Ansprüche 1 bis 4, worin das Bilayer-bildende Amphiphile eine positive Netto-Ladung hat und aus der Gruppe ausgewählt ist, die aus positiv geladenen Formen von Aminodiglycerid, Glyceridcholin, Stearylamin, Trimethylstearylamin and Dioctadecyl-trimethylammonium-Derivaten besteht.

7. Zusammensetzung nach einem der Ansprüche 1 bis 6, worin das Antigen unverteilt ist.

8. Zusammensetzung nach einem der Ansprüche 1 bis 5, worin das Antigen einen HIV-Virus oder eine Fraktion oder ein Fragment davon umfaßt.

9. Zusammensetzung nach einem der Ansprüche 1 bis 5, worin das Antigen die PB1-Fraktion des HIV 1-Virus umfaßt.

10. Zusammensetzung nach einem der Ansprüche 1 bis 7, worin das Antigen ein Peptid umfaßt.

11. Zusammensetzung nach einem der Ansprüche 1 bis 7, worin das Antigen eine Grippevirusfraktion oder ein Fragment davon umfaßt.

**12.** Zusammensetzung nach einem der Ansprüche 1 bis 7, worin das Antigen eine Herpesvirusfraktion, eine Haemophilus B-Bakterienfraktion oder eine Malaria-Sporozoit-Fraktion oder ein Fragment von einer von diesen umfaßt.

**13.** Verfahren zur Herstellung einer Impfstoffzusammensetzung, die ein Adjuvans-Liposom in elektrostatischer Affinitätsassoziierung mit einem außen angeordneten Antigen umfaßt, wobei das Verfahren die Stufe der Kontaktierung in einer wäßrigen Lösung a) eines Liposoms, das ein Bilayer-bildendes Amphiphiles mit einem hydrophilen Teil mit netto-Ladung umfaßt und b) eines Antigens, so daß das Antigen und das Liposom eine elektrostatische Affinitätsassoziierung bilden.

**14.** Verfahren nach Anspruch 13, das weiterhin die Entfernung von nicht-affinitätsassoziiertem Antigen umfaßt.

**15.** Verfahren nach Anspruch 13 oder 14, worin das Antigen unverteilt ist.

**16.** Verfahren nach einem der Ansprüche 13 bis 15, worin das Bilayer-bildende Amphiphile eine positive Netto-Ladung hat und das Antigen einen hydrophilen Teil hat.

**17.** Verfahren nach einem der Ansprüche 13 bis 15, worin das Bilayer-bildende Amphiphile eine negative Netto-Ladung hat und das Antigen einen hydrophilen Teil hat.

**18.** Verfahren nach Anspruch 17, worin das Bilayer-bildende Amphiphile Cholesterin-hemisuccinat ist.

**19.** Verfahren nach Anspruch 17 oder 18, worin das Antigen die PB1-Fraktion des HIV1-Virus umfaßt.

**Patentansprüche für folgende Vertragsstaaten : ES, GR**

**1.** Verfahren zur Herstellung einer Impfstoffzusammensetzung, die ein Adjuvans-Liposom in elektrostatischer Affinitätsassoziierung mit einem außen angeordneten Antigen umfaßt, wobei das Verfahren die Stufe des Kontaktierens in einer wäßrigen Lösung a) eines Liposoms, das ein Bilayer-bildendes Amphiphiles umfaßt, das einen hydrophilen Teil mit Netto-Ladung hat, und b) ein Antigen umfaßt, so daß das Antigen und das Liposom eine elektrostatische Affinitätsassoziierung bilden.

**2.** Verfahren nach Anspruch 1, das weiterhin die Entfernung des nicht-affinitätsassoziierten Antigens umfaßt.

**3.** Verfahren nach Anspruch 1 oder 2, worin das Antigen unverteilt ist.

**4.** Verfahren nach einem der Ansprüche 1 bis 3, worin das Bilayer-bildende Amphiphile eine positive Netto-Ladung hat und das Antigen einen hydrophilen Teil hat.

**5.** Verfahren nach einem der Ansprüche 1 bis 3, worin das Bilayer-bildende Amphiphile eine negative Netto-Ladung hat und das Antigen einen hydrophilen Teil hat.

**6.** Verfahren zur Herstellung einer Zusammensetzung nach Anspruch 1 bis 5, bei dem weiterhin ein zusätzliches Adjuvans hinzugegeben wird.

**7.** Verfahren nach Anspruch 6, worin das Adjuvans Aluminiumhydroxid oder Lipid A umfaßt.

**8.** Verfahren nach einem der Ansprüche 1 bis 7, worin das Bilayer-bildende Amphiphile eine negative Netto-Ladung hat und ausgewählt ist aus der Gruppe, die aus negativ geladenen Formen von Cholesterin-hemisuccinat, Phosphatidylserin, Phosphatidsäure und Phosphytidylglycerin besteht.

**9.** Verfahren nach einem der Ansprüche 1 bis 7, worin das Bilayer-bildende Amphiphile eine positive Netto-Ladung hat und ausgewählt ist aus der Gruppe, die aus positiv geladenen Formen von Aminodiglycerid, Glyceridcholin, Stearylamin, Trimethylstearylamin und Dioctadecyl-trimethylammonium-Derivaten besteht.

**10.** Verfahren nach Anspruch 8, worin das Bilayer-bildende Amphiphile Cholesterin-hemisuccinat ist.

**11.** Verfahren nach einem der Ansprüche 1 bis 9, worin das Antigen einen HIV-Virus oder eine Fraktion oder ein Fragment davon umfaßt.

**12.** Verfahren nach Anspruch 11, worin das Antigen die PB1-Fraktion des HIV 1-Virus umfaßt.

**13.** Verfahren nach einem der Ansprüche 1 bis 10, worin das Antigen ein Peptid umfaßt.

**14.** Verfahren nach einem der Ansprüche 1 bis 10, worin das Antigen eine Grippevirusfraktion oder ein Fragment davon umfaßt.

**15.** Verfahren nach einem der Ansprüche 1 bis 10, worin das Antigen eine Herpesvirusfraktion, eine Haemophilus B-Bakterienfraktion oder eine Malaria-Sporozoit-Fraktion oder ein Fragment von einer von diesen umfaßt.

**Revendications**
**Revendications pour les Etats contractants suivants : AT, BE, CH, DE, FR, GB, IT, LI, LU, NL, SE**

**1.** Composition vaccinale comprenant un liposome adjuvant en association d'affinité électrostatique avec un antigène disposé à l'extérieur, ledit liposome comprenant un agent amphiphile formant une bicouche et ayant une portion hydrophile comportant une charge nette.

**2.** Composition selon la revendication 1, comprenant en outre un adjuvant additionnel.

**3.** Composition selon la revendication 2, dans laquelle ledit adjuvant comprend de l'hydroxyde d'aluminium ou du Lipide A.

**4.** Composition selon l'une quelconque des revendications 1, 2 ou 3, dans laquelle l'antigène est hydrophile.

**5.** Composition selon l'une quelconque des revendications 1 à 4, dans laquelle l'agent amphiphile formant une bicouche a une charge nette négative et est choisi dans le groupe consistant en des formes chargées négativement de cholestérol hémisuccinate, de phosphatidylsérine, d'acide phosphatidique et de phosphatidylglycérol.

**6.** Composition selon l'une quelconque des revendications 1 à 4, dans laquelle l'agent amphiphile formant une bicouche a une charge nette positive et est choisi dans le groupe consistant en des formes chargées positivement d'aminodiglycéride, de glycéridecholine, de triméthylstéarylamine et de dérivés dioctadécyl triméthylammonio.

**7.** Composition selon l'une quelconque des revendications 1 à 6, dans laquelle l'antigène est non-partageant.

**8.** Composition selon l'une quelconque des revendications 1 à 5, dans laquelle l'antigène comprend un virus VIH ou une fraction ou un fragment de celui-ci.

**9.** Composition selon l'une quelconque des revendications 1 à 5, dans laquelle l'antigène comprend la fraction PB1 du virus VIH1.

**10.** Composition selon l'une quelconque des revendications 1 à 7, dans laquelle l'antigène comprend un peptide.

**11.** Composition selon l'une quelconque des revendications 1 à 7, dans laquelle l'antigène comprend une fraction de virus d'influenza ou un de ses fragments.

**12.** Composition selon l'une quelconque des revendications 1 à 7, dans laquelle l'antigène comprend une fraction d'herpèsvirus, une fraction de bactérie d'hémophilie B ou une fraction de sporozoïte de malaria,

14

ou un fragment de l'une quelconque de celles-ci.

13. Méthode pour la production d'une composition de vaccin comprenant un liposome adjuvant en association d'affinité électrostatique avec un antigène disposé à l'extérieur, ladite méthode comprenant l'étape de mise en contact dans une solution aqueuse a) d'un liposome comprenant un agent amphiphile formant une bicouche et ayant une portion hydrophile portant une charge nette, et b) un antigène tels que l'antigène et le liposome forment une association d'affinité électrostatique.

14. Méthode selon la revendication 13, comprenant en outre l'élimination de l'antigène associé sans affinité.

15. méthode selon l'une des revendications 13 ou 14, dans laquelle l'antigène est non-partageant.

16. Méthode selon l'une quelconque des revendications 13 à 15, dans laquelle l'agent amphiphile formant une bicouche a une charge nette positive et l'antigène a une portion hydrophile.

17. Méthode selon l'une quelconque des revendications 13 à 15, dans laquelle l'agent amphiphile formant une bicouche a une charge nette négative et l'antigène a une portion hydrophile.

18. Méthode selon la revendication 17, dans laquelle l'agent amphiphile formant la bicouche est le cholestérol hémisuccinate.

19. Méthode selon l'une des revendications 17 ou 18, dans laquelle l'antigène comprend la fraction PB1 du virus VIH1.

**Revendications pour les Etats contractants suivants : ES, GR**

1. Méthode pour la production d'une composition de vaccin comprenant un liposome adjuvant en association d'affinité électrostatique avec un antigène disposé à l'extérieur, ladite méthode comprenant l'étape de mise en contact dans une solution aqueuse a) d'un liposome comprenant un agent amphiphile formant une bicouche et ayant une portion hydrophile portant une charge nette, et b) un antigène tels que l'antigène et le liposome forment une association d'affinité électrostatique.

2. Méthode selon la revendication 1, comprenant en outre l'élimination de l'antigène associé sans affinité.

3. Méthode selon l'une des revendications 1 ou 2, dans laquelle l'antigène est non-partageant.

4. Méthode selon l'une quelconque des revendications 1 à 3, dans laquelle l'agent amphiphile formant une bicouche a une charge nette positive et l'antigène a une portion hydrophile.

5. Méthode selon l'une quelconque des revendications 1 à 3, dans laquelle l'agent amphiphile formant une bicouche a une charge nette négative et l'antigène a une portion hydrophile.

6. Méthode pour la préparation d'une composition selon l'une quelconque des revendications 1 à 5, comprenant en outre l'addition d'un adjuvant additionnel.

7. Méthode selon la revendication 6, dans laquelle ledit adjuvant comprend de l'hydroxyde d'aluminium ou du Lipide A.

8. Méthode selon l'une quelconque des revendications 1 à 7, dans laquelle l'agent amphiphile formant une bicouche a une charge nette négative et est choisi dans le groupe consistant en des formes chargées négativement de cholestérol hémisuccinate, de phosphatidylsérine, d'acide phosphatidique et de phosphatidylglycérol.

9. Méthode selon l'une quelconque des revendications 1 à 7, dans laquelle l'agent amphiphile formant une bicouche a une charge nette positive et est choisi dans le groupe consistant en des formes chargées positivement d'aminodiglycéride, de glycéridecholine, de triméthylstéarylamine et de dérivés dioctadécyl triméthylammonio.

15

**10.** Méthode selon la revendications 8, dans laquelle l'agent amphiphile formant une bicouche est du cholestérol hémisuccinate.

**11.** Méthode selon l'une quelconque des revendications 1 à 9, dans laquelle l'antigène comprend un virus VIH ou une fraction ou un fragment de celui-ci.

**12.** Méthode selon la revendication 11, dans laquelle l'antigène comprend la fraction PB1 du virus VIH1.

**13.** Méthode selon l'une quelconque des revendications 1 à 10, dans laquelle l'antigène comprend un peptide.

**14.** Méthode selon l'une quelconque des revendications 1 à 10, dans laquelle l'antigène comprend une fraction de virus d'influenza ou un de ses fragments.

**15.** Méthode selon l'une quelconque des revendications 1 à 10, dans laquelle l'antigène comprend une fraction d'herpèsvirus, une fraction de bactérie d'hémophilie B ou une fraction de sporozoïte de malaria, ou un fragment de l'une quelconque de celles-ci.